# EUROPEAN PATENT APPLICATION

(11) **EP 2 287 623 A1**
(43) Date of publication of application: **23.02.2011**
(21) Application number: 09746613.0
(22) Date of filing: 13.05.2009
(51) Int. Cl.: G01N 35/08, C12M 1/00, C12N 15/09, G01N 33/543, G01N 37/00

(54) **MICROANALYSIS CHIP ADHESIVE SHEET, MICROANALYSIS CHIP, AND MANUFACTURING METHOD THEREOF**

(30) Priority: 16.05.2008 JP 2008129102
(71) Applicant: Nippon Kayaku Kabushiki Kaisha, Tokyo 102-8172 (JP)
(72) Inventor: TAKAHASHI, Chihiro, Tokyo 115-8588 (JP); SAKAI, Ryo, Tokyo 115-8588 (JP); YAMADA, Masatoshi, Tokyo 115-8588 (JP)
(74) Representative: Hollatz, Christian
(86) International application number: PCT/JP2009/058892
(87) International publication number: WO 2009/139407

(57) **Abstract**

Disclosed is a microanalysis chip adhesive sheet with an adhesive surface, which can be inexpensively but easily bonded to a substrate at a relatively low temperature, with which fabrication of analysis chips is simplified, and which has the capability of fixing a selective bonding substance to the sheet surface. The microanalysis chip is **characterized in that** a microanalysis chip adhesive sheet, which comprises a plastic sheet provided with an adhesive layer formed from an adhesive that contains a polymer in which monomer structural units that contain carboxyl radicals or acid anhydride radicals constitute 5-25% by weight of the [total] monomer structural units in the polymer, and a substrate, which has protrusions and recesses on the surface thereof, are bonded together so that the surface of the substrate having the projections and concavities and the adhesive layer of the aforementioned microanalysis chip adhesive sheet are on the inside. Gaps at the concave parts of the substrate between the substrate and the adhesive layer of the aforementioned microanalysis chip adhesive sheet form a flow path, a part of which is the aforementioned adhesive layer, and a selective bonding substance is fixed to the adhesive layer surface.

## Description

### Technical Field

The present invention relates to a microanalysis chip useful for analyses of extremely trace components, and a method of manufacturing such a microanalysis chip.

### Background Art

Recently, attention has been focused on biochips, which are a device having a bioactive substance fixed to a solid phase substrate, as means for realizing high throughput operations in drug discovery studies or clinical studies. Typical examples of the bioactive substances to be fixed include nucleic acids, proteins, antibodies, sugar chains, glycoprotein and aptamer. In particular, many commercial products of nucleic acid microarrays, which are biochips having a fixed nucleic acid, are already available on the market. The chips are of the form having various bioactive substances spotted and fixed on a flat substrate, and this type is mainly used for studies and analyses in research institutes.

More recently, studies have been underway briskly on chemical reactions or separation or microminiaturization of analytical systems utilizing micromachining technology called microanalysis chips, µTAS (micro total analytical system) or lab on chips, and it has become possible to conduct various chemical reactions, particularly physiological reactions on a microchannel (fine flow path). This system is capable of rapidly analyzing a trace sample, and thus commercialization of the next generation biochips taking advantage of this feature, particularly diagnostic chips in medical institutes is expected and is receiving attention. (Hereinafter, these systems are generically termed microanalysis chips.)

Presently, common biochips or microanalysis chips are glass-made ones. To make a microanalysis chip with a glass substrate, there is for instance a method according to which a substrate is coated with a metal and a photoresist resin and after transferring a microchannel pattern, etching is carried out. Glass, however, is unsuited for mass production and also very high in cost, and thus conversion of glass to plastic is desired.

Plastic-made biochips or microanalysis chips can be produced by various molding methods such as injection molding by using various plastic materials. In injection molding, a molten thermoplastic material is introduced into a mold cavity, and the cavity is cooled to harden the resin, whereby it is possible to produce a chip substrate efficiently and economically. Therefore, plastic biochips and microanalysis chips are suited for mass production. These plastic biochips and microanalysis chips, however, still have many disadvantages technically, and sufficient technical know-how to substitute the glass-made chips with plastic ones has not been obtained yet. In particular, not only plastic biochips and microanalysis chips but also glass biochips and microanalysis chips (biochips and microanalysis chips are generically called microfluidics and featured by provision of a fine flow path in the inside of the chip) have still many defects, and they are still in the stage of development. In particular, a problem of the plastic microanalysis chips, in which it is necessary to bond a different plastic plate on the plastic plate having a fine flow path to thereby cover the flow path, is that an inexpensive, simple and reliable system for bonding has not been found yet. This is one of the large factors that obstruct practical utilization of plastic microanalysis chips.

In the bonding step in the manufacture of plastic biochips or microanalysis chips, there is mainly employed a system utilizing hot bonding by use of heat, supersonic waves or laser, or a system using an organic adhesive (Patent Literature 1). Fusion by heating tends to give rise to the problem of deactivation described later of bioactive substance due to heating. In the case of immunoanalysis which makes use of an antigen-antibody reaction, it is possible to know the presence of a trace substance to be analyzed in a sample and its concentration by conducting measurement with a thermal lens microscope or other means. However, heating tends to cause deformation of the inside of the fine flow path or degradation of surface quality of the flow path surface, making it difficult in some cases to conduct measurement. In the case of heat bonding by supersonic waves, although it is possible to bond the area of several millimeter square, this method is unsuited for heat bonding of the area of several centimeter square and tends to cause insufficient bonding. In the case of laser irradiation, although there may be no problem at the irradiated area, it is extremely difficult to heat only the central portion of plastic, such as the bonded areas of two plastic sheets. This method also involves the problem of elevated cost of equipment.

The bonding method by use of an organic adhesive is very effective as it is capable of bonding together the plastic sheets at a relatively low temperature. However, in the plastic sheets obtained from, for instance, injection molding, depressions and projections of the order of several µm to several 10 µm are formed at the part which should normally remain flat, due to sink marks during molding or for other causes, and thus it needs to fill up the depressions and projections so as to well bond the plastic sheets to each other with an adhesive. To attain this, an adhesive thickness of the order of several ten µm is required. When bonding is made with an adhesive having a thickness of the order of 10 µm, there tends to ooze out a surplus adhesive from between the substrates, which likely causes blockage of microchannels or contamination of the inner walls. In particular, in the case of bonding with a thermosetting type adhesive, there tends to arise the problem of deactivation of the bioactive substance as in the case of heat bonding system. Also, there is possibility of hampering measurement in the thermal lens microscope method.

Regarding bonding of plastic products, which are not limited to biochips and microanalysis chips, there is a proposal of a system other than one described above in which a protrusion is provided at a part of the bonding surface of the object to be bonded, and is fitted in another area to be joined, and this portion is heat fused by supersonic vibrations (Patent Literature 2). Use of this system, however, is limited to not so fine, relatively large molded products, and the biochips and microanalysis chips having a fine structure are not included in the ambit of application of this system.

Further, when considering application to analytical chips, particularly biochips, there are many cases where various types of substances, particularly nucleic acid, protein, antibody, sugar chain, glycoprotein or aptamer is coated or fixed to the site of detection. These bioactive substances are vulnerable to heat and liable to be chemically deactivated when heated, and thus the bonding processes involving application of high temperatures are not suited for the manufacture of biochips and microanalysis chips.

As a method for providing a plastic microanalysis chip which can be inexpensively, easily, firmly and securely bonded to a substrate at a relatively low temperature, there has been proposed a method in which a plastic substrate having a fine flow path on the surface and a plastic film are bonded with the interposition of an ultraviolet curing adhesive on a surface side having a fine flow path (Patent Literature 3). Patent Literature 3, however, is totally silent to the concrete method of fixing of a selective bonding substance to the inside surface of a cover member.

In these microanalysis chips, importance is attached not only to simplification of the manufacturing process but also to quantitative quality, precision of analysis and economy. The sample packed in a microanalysis chip is usually very small in quantity, and thus the substance which becomes the object of analysis exists in a trace quantity in many cases. A microanalysis chip is required to technically cope with the situations so that it can analyze a trace substance at high sensitivity with high precision.

One of the factors which exert large influence on the improvement of detection sensitivity is the fixing efficiency of the selective bonding substance on the fine flow path, namely, the ability to fix the selective bonding substance on the fine flow path. Analytical sensitivity is obviously hampered when the quantity of the selective bonding substrate that can be fixed to not only the substrate forming a fine flow path but also the inside surface of the cover member is small.

### Citation list

### Patent Literature

Patent Literature 1: Japanese Patent Laid-Open No. 2002-139419
Patent Literature 2: Japanese Patent Laid-Open No. 5-16241
Patent Literature 3: Japanese Patent Laid-Open No. 2007-240461

### Summary of Invention

### Technical Problem

The present invention, which has been made in view of the above circumstances, is intended to provide a microanalysis chip inexpensively but easily at a relatively low temperature by using, as a microanalysis chip substrate or a coated substrate, an adhesive sheet whose sheet surface has adhesiveness and which can fix a selective bonding substance to the sheet surface, and to provide a microanalysis chip which enables rapid processing and high precision detection.

### Solution to Problem

As a result of intense studies for solving the above-mentioned problems, the present inventors have found that a microanalysis chip and its adhesive sheet can be obtained which can be bonded rapidly with a satisfactory bonding strength at a relatively low temperature without causing blockage of the fine flow path and has a capability of fixing a selective bonding substance to a surface of the adhesive layer. This can be realized by bonding with an adhesive a substrate having depressions and projections which serves as a fine flow path on the surface and a plastic sheet having an adhesive layer on the surface which functions as a covering substrate. It has been confirmed that a biochip or a microanalysis chip worked out by utilizing the above-described technology is obtainable, and this has led to the attainment of the present invention.

Thus, the present invention provides:
(1) A microanalysis chip adhesive sheet comprising a plastic sheet having an adhesive layer formed from a crosslinkable adhesive containing a polymer in which monomer structural units containing carboxyl groups or acid anhydride groups constitute 5 to 25% by weight of the total monomer structural units in the polymer;
(2) A microanalysis chip adhesive sheet according to (1), wherein a selective bonding substance is fixed to the surface of the adhesive layer;
(3) A microanalysis chip adhesive sheet according to (2), wherein the selective bonding substance is fixed by binding carboxyl groups or acid anhydride groups on the surface of the adhesive layer with functional groups of the selective bonding substance;
(4) A microanalysis chip adhesive sheet according to (2) or (3), wherein the selective bonding substance is any of a nucleic acid, a protein, a peptide, a saccharide and a lipid;
(5) A microanalysis chip adhesive sheet according to any one of (1) to (4), wherein the plastic sheet is made of any of a polyester, a cellulose derivative and a polycarbonate;
(6) A microanalysis chip prepared by bonding together a substrate having depressions and projections on a surface and a microanalysis chip adhesive sheet according to any one of (1) to (5) so that the surface having depressions and projections of the substrate and the adhesive layer of the adhesive sheet are placed on the inside;
(7) A microanalysis chip according to (6), wherein a gap is formed between the adhesive layer and the substrate;
(8) A microanalysis chip according to (6) or (7), wherein the substrate having depressions and projections on the surface is made of plastic;
(9) A method of manufacturing a microanalysis chip according to any one of (6) to (8), which comprises the steps of: applying an adhesive onto a plastic sheet; and roll laminating the adhesive-applied plastic sheet and a substrate having depressions and projections on a surface so that the surface having depressions and projections of the substrate and the adhesive are placed on the inside; and
(10) A method of manufacturing a microanalysis chip according to any one of claims 6 to 8, which comprises the steps of: applying an adhesive onto a plastic sheet to form an adhesive layer; fixing a selective bonding substance on the surface of the adhesive layer; and roll laminating the plastic sheet having the adhesive layer on which the selective bonding substance is fixed and a substrate having depressions and projections on a surface so that the surface having depressions and projections of the substrate and the adhesive layer are placed on the inside.

### Effects of Invention

The microanalysis chip adhesive sheet according to the present invention is featured in that the sheet surface has adhesiveness, and that the sheet has a capability to fix a selective bonding substance to the sheet surface. Such a product is suited as a covering sheet for a microanalysis chip. By use of the microanalaysis chip adhesive sheet of the present invention, it is possible to easily provide microanalysis chips at low cost and at a relatively low temperature, which enables rapid processing and high-precision detection.

### Embodiments for Carrying Out the Invention

The present invention will be described below in detail with reference to its embodiments.

The present invention relates to a microanalysis chip adhesive sheet having a plastic sheet provided with an adhesive layer formed from an adhesive that contains a polymer in which the monomer structural units containing carboxyl groups or acid anhydride groups constitute 5 to 25% by weight of the total monomer structural units in the polymer; a microanalysis chip; and a manufacturing method thereof. The present invention is mainly targeted at a microanalysis chip having a fine flow path. "Fine flow path" in the present invention designates a fine covered groove formed with an intention to flow a liquid substance such as water and organic solvents. Such a flow path preferably has a width of 1000 µm or less and a depth of 500 µm or less.

The adhesive used in the present invention is a copolymer whose monomers include a monomer containing at least one carboxyl group or at least one acid anhydride group. The copolymer is preferably one obtained by copolymerizing a vinyl derivative as a main monomer having no functional group and a monomer containing at least one carboxyl group or at least one acid anhydride group, and more preferably one mainly containing an acrylic adhesive. The acrylic adhesive is a copolymer of a (meth)acrylic alkyl ester as a main monomer having no functional group and a (meth)acrylic acid monomer or an unsaturated polybasic acid. The (meth)acrylic acid monomer and unsaturated polybasic acid function as a reaction point in the case where a crosslinking agent is used or a reaction point of the selective bonding substance.

Examples of the (meth)acrylic alkyl ester having no functional group include acrylic alkyl esters such as methyl acrylate, ethyl acrylate, propyl acrylate, butyl acrylate, amyl acrylate, 2-ethylhexyl acrylate, octyl acrylate, cyclohexyl acrylate or benzyl acrylate, and methacrylic alkyl esters such as methyl methacrylate, ethyl methacrylate, butyl methacrylate, 2-ethylhexyl methacrylate, cyclohexyl methacrylate or benzyl methacrylate. Other vinyl derivative monomers usable in this invention include vinyl acetate, styrene or acrylonitrile. Butyl acrylate or 2-ethylhexyl acrylate, particularly butyl acrylate is preferred for use as the vinyl derivative monomer in this invention.

Examples of the monomer containing at least one carboxyl group or at least one acid anhydride group include acrylic acids, methacrylic acids, maleic acid, itaconic acid, β-carboxyethyl acrylate, ω-carboxy-polycaprolactone monoacrylate, 2-acryloyloxyethyl phthalate, 2-acryloyloxypropyl phthalate and 2-acryloyloxyethylhexahydrophthalate. Acrylic acid or methacrylic acid is preferably used as the monomer containing at least one carboxyl group or at least one acid anhydride group.

The reasons for preference of these materials are excellent bonding and cohesive forces, high stability with respect to light and oxygen because of absence of unsaturated bonds in the polymer, and obtainability of any quality and characteristics according to the purpose of use by selection of the type and molecular weight of the polymer.

It is essential that the monomer structural units containing carboxyl groups or acid anhydride groups constitute 5 to 25% by weight of the total monomer structural units in the polymer. This ratio preferably stays in a range of 10 to 20% by weight. If the ratio is lower than 5% by weight, the amount of functional groups (carboxyl groups or acid anhydride groups) decreases and the amount of bonding of the selective bonding substance becomes insufficient. If the above-said ratio is higher than 25% by weight, the adhesive layer becomes liable to separate or dissolve when immersed in a buffer solution (pH 6-8) in the step of activating carboxyl groups on the adhesive sheet surface. Thus, in the above-defined range of monomer structural units ratio, there can be obtained an adhesive layer which is resistant to separation or dissolving even when immersed in a buffer solution (pH 6-8) in the step of activating carboxyl groups on the adhesive sheet surface, and can maintain a sufficient amount of surface carboxyl groups as well as a sufficient amount of bonding of the selective bonding substance.

As the adhesive in the present invention, a crosslinkable type is used. In the case of crosslinkable type, there is employed, for example, a method using various types of crosslinkable agent such as epoxy compounds, isocyanate compounds, metal chelate compounds, metal alkoxides, metal salts, amine compounds, hydrazine compounds or aldehyde compounds, or a method using irradiation. Either of these methods is properly selected depending on the type of functional groups and other factors.

The degree of crosslinking of the polymer of the adhesive differs depending on the various conditions such as the type and composition of the adhesive (adhesive composition) and is not specifically defined, but usually it is preferably within the range of 10 to 95%, and more preferably 15 to 90% in terms of sol-gel process fraction. If the degree of crosslinking is lower than 10%, the adhesive layer becomes liable to separate or dissolve when immersed in a buffer solution (pH 6-8)in the step of activation of carboxyl groups on the adhesive sheet surface. If the degree of crosslinking is higher than 95%, viscosity stability of the coating solution for forming the adhesive layer is bad, which may cause worsening of workability, decrease of the amount of functional groups (carboxyl groups) or insufficient amount of bonding of the selective bonding substance. Thus, by keeping the degree of crosslinking within the above-defined range, there can thus be obtained an adhesive layer having the following advantages. That is, the adhesive layer hardly separates or dissolves when immersed in a buffer solution (pH 6-8) in the step of activating carboxyl groups on the adhesive sheet surface. Also, workability is good, and the amount of carboxyl groups on the surface is sufficient, and the amount of bonding of the selective bonding substance is sufficient.

The adhesive may contain a plastisizer as required. As the plastisizer, esters such as phthalic acid esters, trimellitic acid esters, pyromellitic acid esters, adipic acid esters, sebacic acid esters, phosphoric triesters or glycol esters; process oil, liquid polyether, liquid polyterpene and other liquid resins can be used either singly or as a mixture of two or more of them. The plasticizer is preferably one having good compatibility with the adhesive used.

Also, the adhesive may contain, beside the plasticizer, various additives such as an ultraviolet absorbent or an antioxidant.

The adhesive coating method is not specified in this invention; it is possible to use various types of coating devices such as a comma coater, a bar coater, a spin coater, a spray coater, a roll coater, a gravure coater, and a knife coater.

The adhesive layer thickness is preferably in the range of 1 to 50 µm, and more preferably 1 to 10 µm. If the adhesive thickness is below the lower limit of the above-defined range, sufficient adhesive strength cannot be obtained, and also air cells tend to get into the system during bonding. If the adhesive thickness exceeds the upper limit of the above-defined range, resin tends to get into the fine flow path during bonding, causing blockage of the flow path.

The plastic used as the base material for the plastic sheet in the present invention is a polymer having a melting point and Tg, for instance, high-density polyethylene, low-density polyethylene, polypropylene, polystyrene, various cyclic polyolefins and acrylic resin such as polymethyl methacrylate, polynorbornene, polyphenylene oxide, polycarbonate, polyamide, polyester, half-cured phenol resin, half-cured epoxy resin, various types of thermoplastics and cellulose derivative. No specific definition is made in the present invention on the type and other properties of the polymer used, such as polymerization degree, melting point, Tg and modulus of elasticity, but polyester, acrylic resin such as polymethyl methacrylate, polystyrene, cellulose derivative or polycarbonate is preferred, with polyester, cellulose derivative or polycarbonate being more preferred for use as the polymer in the present invention.

In the present invention, "selective bonding substance" designates various materials which can selectively bond to the target material either directly or indirectly. Typical examples of the selective bonding substance that can be bonded to the substrate surface are nucleic acids, proteins, peptides, saccharides or lipids.

Nucleic acids may be DNA or RNA, or may be PNA. Single chain nucleic acids having a specific base sequence are selectively hybridized with another single chain nucleic acid having a base sequence complementary with the base sequence or a part thereof, and thus these single chain nucleic acids fall within the concept of "selective bonding substance" designated in the present invention.

The nucleic acids used in the present invention may be those derived from natural products such as living cells, or may be those synthesized by a nucleic acid synthesizer. Conventional methods can be used for preparing DNA or RNA from the living cells. For instance, for the extraction of DNA, Blin et al method (Nucleic Acids Res. 3:2303 (1976)) is available, and for the extraction of RNA, Favaloro et al method (Methods Enzymol. 65:718 (1980)) is instructive. Further, as the nucleic acids to be fixed, it is possible to use chain or cyclic plasmid DNA or chromosome DNA, the DNA fragments obtained by cutting these plasmid DNA or chromosome DNA with a restriction enzyme or chemical means, DNA or RNA which is synthesized emzymatically in a test tube, or chemically synthesized oligonucleotide.

As the protein, there can be used antibodies, antigen binding fragments of antibodies such as Fab or F(ab')₂ fragments, and various sorts of antigen. Antibodies and their antigen binding fragments selectively bond to the corresponding antigens, while antigens also selectively bond to the corresponding antibodies, and thus they are included within the category of "selective bonding substance."

Examples of the peptides usable in the present invention include peptides which are produced in vivo such as a ribosomal peptide, a non-ribosomal peptide or a digestive peptide, and synthetic peptides.

The saccharides usable in the present invention include various monosaccharides and sugar chains such as oligosaccharides and polysaccharides.

The lipid may be either simple lipid or complex lipid.

It is also possible to fix the substances having antigenicity other than the above-mentioned nucleic acids, proteins, peptides, saccharides or lipids. Cells may be fixed to the substrate surface as a selective bonding substance.

Among these selective bonding substances, especially preferred for use in this invention are DNA, RNA, proteins, peptides, sugar, sugar chains and lipids.

Examples of a method of bonding the selective bonding substance to be fixed to the adhesive surface in the present invention include known reactions capable of generating chemical bonds such as condensation reaction, addition reaction and substitution reaction, but dehydrative condensation reaction or substitution reaction which produces amide bonds or ester bonds is preferred. In particular, a dehydrative condensation reaction which forms amide bonds from carboxyl groups and amino groups or a condensation reaction which forms amide bonds from acid anhydride and amino groups is preferred. Ordinary peptide condensation reaction may be employed for the dehydrative condensation reaction. As the dehydrative condensing agent for the above reactions, there may be used, for instance, carbodiimdes such as dicyclohexylcarbodiimide, diisopropylcarbodiimide, 1-dimethylaminopropyl-3-ethylcarbodiimide and 1-methyl-3-(3-dimethylaminopropyl)carbodiimide, phosphonium salts such as benzotriazole-1-yl-tris(dimethylamino)phosphonium hexafluorophosphate, and diphenylphospholylazide. Of these compounds, 1-methyl-3-(3-dimethylaminopropyl)carbodiimide is preferred. The amount of such a dehydrative condensing agent is 0.5 to 10 molar equivalents, and preferably 1 to 2 molar equivalents to carboxyl groups. The reaction is carried out in the presence or absence of an additive. As the additive, N-hydroxysuccinimide, 1-hydroxybenzotriazole, 4-nitrophenol, pentafluorophenol or the like can be used. In the case where the additive is used, the amount thereof is about 0.5 to 10 molar equivalents, and preferably about 1 to 2 molar equivalents to carboxyl groups.

The adhesive sheet of the present invention may have a release sheet on the adhesive layer. A polyester film, for instance, may be used as the release sheet.

The adhesive sheet obtained in the manner described above is subjected to seasoning as required.

The substrate having depressions and projections used in the present invention may be made of plastic, glass, silicon, metal, ceramic or the like. The plastic materials usable in the present invention include high-density polyethylene, low-density polyethylene, polypropylene, polystyrene, various types of cyclic polyolefins, polymethyl methacrylate, polynorbornene, polyphenylene oxide, polycarbonates, polyamides, polyesters, half-cured phenol resins, half-cured epoxy resins, and various types of thermoplastics. Of these materials, polymethyl methacrylate, polycarbonate, polystyrene, cyclic polyolefin or polyethylene terephthalate is preferred because of good transparency which is advantageous for observation and measurements. The types of glass usable as the substrate material in the present invention include quartz glass, non-alkali glass, borosilicate glass, or soda-lime glass, of which quartz glass and borosilicate glass are preferred because of good processability. The metals usable for the substrate include silver, nickel, aluminum alloys, stainless steel, Hastelloy or titanium. The substrate may be made of a single material or a combination of two or more different materials. In the latter case, for instance, a photosensitive resin is applied onto a glass or silicon substrate, and the light-irradiated portion or non-irradiated portion alone is selectively removed, thereby forming the raised and depressed portions of plastic on the glass or silicon substrate. This method is not the only available method. A negative resist or a positive resist can be used as the photosensitive resin composition, but epoxy resin type negative resist is preferred as it is capable of providing a sufficient film thickness and has good processability. Typical examples of such a negative resist are SU-8 (manufactured by MicroChem Corporation) and SU-8 3000 (manufactured by Kayaku MicroChem Corporation)

As the substrate material, plastic or glass is preferred in view of processability and advantage for observation, but plastic is most preferred because of good adhesion to the microanalysis chip adhesive sheet.

Fine depressions and projections can be formed by such methods as etching, photolithography, injection molding, hot press, imprinting, molding, electrospark machining, cutting, sandblasting or stereo lithography. If necessary, two or more of these methods may be combined. The shape of depressions and projections is usually groove-like, circular or rectangular hole- or pillar-like. The configuration of the side walls of the groove, etc., is usually vertical, but it may also take a taper, back taper or arc shape. Regarding the size of depressions and projections, in the case of a groove, it is usually 10 to 1,000 µm in depth and 10 to 1,000 µm in width, and in the case of a hole- or pillar-like shape, the diameter of its circle or one side of its rectangle is 10 to 1,000 µm. The shape and size of depressions and projections and their side walls have been shown above by way of example, but they may differ greatly depending on the working method employed as well as the objective function of the product. Also, the microanalysis chips are usually made in various shapes and sizes, and thus the above statements on the shape and size are not definitive.

In the present invention, a substrate having depressions and projections and a microanalysis chip adhesive sheet are bonded to each other in such a way that the surface of the substrate having depressions and projections and the adhesive layer of the adhesive sheet are placed on the inside. In this case, gaps at the depressed parts of the substrate constitute a microanalysis chip functional section such as a flow path, chamber and mixer. The raised parts are bonded to the adhesive layer of the microanalysis chip adhesive sheet to prevent leakage of liquid and other troubles. The raised parts are preferably uniform in height so as to enhance adhesion to the microanalysis chip adhesive sheet.

Bonding of the substrate having depressions and projections and the microanalysis chip adhesive sheet in the present invention is performed under heating and pressure using a roll or a plate under atmospheric pressure or in vacuum. In this operation, if necessary, one or both of the microanalysis chip adhesive sheet and the substrate may be fixed to a jig. In view of the operating time of the system and durability of the microanalysis chip adhesive sheet and the substrate, it is preferable to confine the degree of vacuum for the operation within the range of from atmospheric pressure to 10 Pa, the heating temperature within the range of 20 to 100°C, and the pressure within the range of 0.01 to 10 MPa.

Carboxyl groups or acid anhydride groups of the adhesive sheet or microanalysis chip formed as described above are activated in use.

The microanalysis chip manufacturing method of the present invention is characterized in that it enables bonding to be firmly made over a relatively wide area at a relatively low temperature without causing contamination, and in that thus the product of the present invention can offer a high-performance plastic biochip or microanalysis chip. In particular, it can be applied favorably to products which have undergone fine working such as microfluidics. In particular, noteworthy is a group of products in which a bioactive substance is fixed on the surface of the chip or the inside, such as a nucleic acid chip, a protein chip, an antibody chip, an aptamer chip or a sugar protein chip.

### Examples

The present invention will be specifically described below with reference to Examples, but the present invention is not limited in any way by these Examples.

A polyester film was used as a substrate. The adhesive layers were formed by using the adhesives of the following compositions to make the adhesive sheets.

### Example 1 (Adhesive sheet preparation 1)

### Method of synthesizing polymer A

127.5 g of n-butyl acrylate and 22.5 g of acrylic acid were dissolved in 225 g of ethyl acetate, to which 0.05 g of azobisisobutyronitrile was added. The mixture was polymerized at 90°C for 6.5 hours to obtain an acrylic resin copolymer (weight average molecular weight: Mw = 800,000) (polymer A). This product was diluted with ethyl acetate and methyl ethyl ketone to obtain an acrylic resin copolymer (polymer A) solution with a solid fraction of 26.7% and a viscosity of 6,500 mPas.

100 parts by weight of the polymer A solution (butyl acrylate/acrylic acid = 85/15), 0.02 parts by weight of an epoxy type crosslinking agent Tetrad-X (manufactured by Mitsubishi Gas Chemical Company, Inc.) and 570 parts by weight of methyl ethyl ketone were mixed homogeneously so that solid matter was dissolved in liquid. Thus, an adhesive composition was prepared, and was comma coated on a polyester film (whose one side had been subjected to easy adhering treatment, A4100)(thickness: 100 µm) and dried to form an adhesive layer (5 µm thick). This adhesive layer was covered with a polyester film as a release sheet (having its one side treated with silicone) (AL5, manufactured by Lintec Corporation, thickness: 38 µm). The obtained sheet was subjected to seasoning at 35°C for 7 days to make an adhesive sheet.

### Example 2 (Adhesive sheet preparation 2)

An adhesive sheet was made in the same way as in Example 1 except that the epoxy type crosslinking agent Tetrad-X (manufactured by Mitsubishi Gas Chemical Company, Inc.) was added in an amount of 0.05 parts by weight.

### Comparative Example 1 (Adhesive sheet preparation 3)

### Method of synthesizing polymer B

105 g of n-butyl acrylate and 45 g of acrylic acid were dissolved in 225 g of ethyl acetate, to which 0.05 g of azobisisobutyronitrile was added. The mixture was polymerized at 90°C for 6.5 hours to obtain an acrylic resin copolymer (weight average molecular weight: Mw =700,000)(polymer B). This product was diluted with ethyl acetate and methyl ethyl ketone to obtain a acrylic resin copolymer (polymer B) solution having a solid fraction of 27.7% and a viscosity of 57,000 mPas.

An adhesive sheet was made in the same way as in Example 1 except that 100 parts by weight of polymer B solution (weight average molecular weight (Mw) = 700,000; solid fraction: 27.7%; viscosity: 57,000 mPas)(butyl acrylate/acrylic acid = 70/30) was used as the adhesive composition.

### Comparative Example 2 (Adhesive sheet preparation 4)

An adhesive sheet was made in the same way as in Comparative Example 1 except that the epoxy type crosslinking agent Tetrad-X (manufactured by Mitsubishi Gas Chemical Company, Inc.) was added in an amount of 0.05 parts by weight.

### Comparative Example 3 (Adhesive sheet preparation 5)

An adhesive sheet was made in the same way as in Example 1 except that no epoxy type crosslinking agent Tetrad-X (manufactured by Mitsubishi Gas Chemical Company, Inc.) was added.

### Comparative Example 4 (Adhesive sheet preparation 6)

An adhesive sheet was made in the same way as in Comparative Example 1 except that no epoxy type crosslinking agent Tetrad-X (manufactured by Mitsubishi Gas Chemical Company, Inc.) was added.

pH resistance and protein (trypsin) binding capacity of the adhesive sheets of Examples 1 and 2 and Comparative Examples 1 to 4 were evaluated. Results of evaluation are shown in Table 1.

### Method of evaluating pH resistance

2-Morpholineethanesulfonic acid (MES, manufactured by Dojindo Laboratories) was dissolved in distilled water so as to have a final concentration of 0.5 M, and then a 1N sodium hydroxide solution was added dropwise to make pH 6.0 to 7.0 to prepare a MES buffer solution. Separately from the above, 2-amino-2-(hydroxymethyl)propane-1,3-diol (Tris, manufactured by Nacalai Tesque, Inc.) was dissolved in distilled water to have a final concentration of 0.5 M, and then 1N hydrochloric acid was added dropwise to make pH 7.0 to 8.0 to prepare a Tris buffer solution. In use, each of these buffer solutions was diluted to 0.1 M with distilled water.

Each of the adhesive sheets, cut to a size of 20 mm x 20 mm, was immersed in 4 ml of each of the diluted buffer solutions at room temperature for 20 minutes, and the condition of the adhesive layer was observed visually.
A: No change under all conditions of pH.
B: Adhesive layer dissolved, peeled or swelled under certain conditions of pH.
C: Adhesive layer dissolved, peeled or swelled under all conditions of pH.

### Method of evaluating protein (trypsin) binding capacity

0.5 M sodium chloride was added to a 0.1 M 2-morpholineethanesulfonic acid buffer solution (MES, pH 6.0, manufactured by Dojindo Laboratories) to form a reaction solution A, and each adhesive sheet cut to a size of 20 mm x 20 mm was immersed in 2 ml of the reaction solution A at room temperature for 20 minutes. 0.8 mg of 1-ethyl-3-(3-dimethylaminopropyl)carbodiimide (EDC, manufactured by Pierce, Inc.) and 2.2 mg of N-hydroxysulfosuccineimide (Sulfo-NHS, manufactured by Pierce, Inc.) were added to the reaction solution A in which the adhesive sheet was immersed, and the mixed solution was kept at room temperature for 60 minutes to thereby activate carboxyl groups of the adhesive sheet. The adhesive sheet having carboxyl groups that had been activated was washed twice with the reaction solution A. Two mg of trypsin (manufactured by Roche) was added to 2 ml of the reaction solution A, and the adhesive sheet was immersed in this solution, with the reaction carried out at room temperature for 3 hours to have trypsin bound to the adhesive sheet. On conclusion of the reaction, the adhesive sheet was taken out of the solution, and the amount of residual trypsin in the reaction solution was measured using bovine serum albumin (BSA, Pierce) as standard material by a BSA Protein Assay Kit (manufactured by Pierce, Inc.), thereby determining the amount of protein binding to the adhesive sheet.

**[Table 1]**

| | pH resistance (pH6-8) | Amount of protein binding (µg/cm²) |
|---|---|---|
| Example 1 | A | 43 |
| Example 2 | A | 19 |
| Comparative Example 1 | C | - |
| Comparative Example 2 | C | - |
| Comparative Example 3 | B | 38 |
| Comparative Example 4 | C | - |

Examples 1 and 2 showed high pH resistance and a sufficient amount of protein binding. On the other hand, Comparative Examples 1, 2 and 4 were unsatisfactory in pH resistance and incapable of determining protein binding capacity. Comparative Example 3 was narrow in tolerable range of pH conditions and limited in the conditions for the activation step.

### Examples 3 and 4 (Microanalysis chip manufacture 1 and 2)

An epoxy resin type negative resist SU-8 3050 (manufactured by Kayaku MicroChem Corporation) was spin coated on a silicon substrate at 3,000 rpm and hot dried using a hot plate at 95°C for 20 minutes to obtain a 50µ m thick homogeneous coating film. This coating film was exposed to light at 250 mJ/cm² through a mask having a 100 µm wide flow path pattern using a mask aligner (MA-20 manufactured by Mikasa Co., Ltd.). The coating film was baked at 95°C for 6 minutes using a hot plate and then developed with a developer to obtain a substrate having a 100 µm wide flow path pattern. This substrate was laminated with a microanalysis chip adhesive sheet obtained in Examples 1 and 2 from which the release sheet had been removed so that the flow path pattern and the adhesive layer were placed on the inside, thereby obtaining a microanalysis chip. Holes were formed with a needle at both ends of the flow path, and a tube was connected thereto, and pure water was supplied by a syringe pump. No peel or leakage at the joint of the substrate and the microanalysis chip adhesive sheet was observed, and it was confirmed that this product can well function as a microanalysis chip.

## Claims

1. A microanalysis chip adhesive sheet comprising a plastic sheet having an adhesive layer formed from a crosslinkable adhesive containing a polymer in which monomer structural units containing carboxyl groups or acid anhydride groups constitute 5 to 25% by weight of the total monomer structural units in the polymer.

2. The microanalysis chip adhesive sheet according to claim 1, wherein a selective bonding substance is fixed to the surface of the adhesive layer.

3. The microanalysis chip adhesive sheet according to claim 2, wherein the selective bonding substance is fixed by binding carboxyl groups or acid anhydride groups on the surface of the adhesive layer with functional groups of the selective bonding substance.

4. The microanalysis chip adhesive sheet according to claim 2 or 3, wherein the selective bonding substance is any of a nucleic acid, a protein, a peptide, a saccharide and a lipid.

5. The microanalysis chip adhesive sheet according to any one of claims 1 to 4, wherein the plastic sheet is made of any of a polyester, a cellulose derivative and a polycarbonate.

6. A microanalysis chip prepared by bonding together a substrate having depressions and projections on a surface and a microanalysis chip adhesive sheet according to any one of claims 1 to 5 so that the surface having depressions and projections of the substrate and an adhesive layer of the adhesive sheet are placed on the inside.

7. The microanalysis chip according to claim 6, wherein a gap is formed between the adhesive layer and the substrate.

8. The microanalysis chip according to claim 6 or 7, wherein the substrate having depressions and projections on the surface is made of plastic.

9. A method of manufacturing a microanalysis chip according to any one of claims 6 to 8, which comprises the steps of: applying an adhesive onto a plastic sheet; roll laminating the adhesive-applied plastic sheet and a substrate having depressions and projections on a surface so that the surface having depressions and projections of the substrate and the adhesive are placed on the inside.

10. A method of manufacturing a microanalysis chip according to any one of claims 6 to 8, which comprises the steps of: applying an adhesive onto a plastic sheet to form an adhesive layer; fixing a selective bonding substance on the surface of the adhesive layer; and roll laminating the plastic sheet having the adhesive layer on which the selective bonding substance is fixed and a substrate having depressions and projections on a surface so that the surface having depressions and projections of the substrate and the adhesive layer are placed on the inside.
